# EUROPEAN PATENT SPECIFICATION

(11) **EP 1 932 547 B1**
(45) Date of publication and mention of the grant of the patent: **13.07.2011**
(21) Application number: 08003478.8
(22) Date of filing: 09.03.2005
(51) Int. Cl.: A61L 15/58, A61L 15/22

(54) **Extended stay-on wound dressing**
Wundverband mit verlängerter Haftung
Pansement restant étendu

(30) Priority: 14.06.2004 US 867388
(43) Date of publication of application: 18.06.2008
(62) Divisional of application: 05725080.5
(73) Proprietor: Jentec, Inc., Northvale, NJ 07647 (US)
(72) Inventor: Jensen, Jarl, B., New York New York 10960 (US)
(74) Representative: Grünecker, Kinkeldey, Stockmair & Schwanhäusser Anwaltssozietät

(56) References cited:
- EP-A- 1 020 195
- EP-A- 1 020 198
- WO-A-01/05340
- WO-A-99/54422
- WO-A-02/066087

## Description

The present invention relates to the field of dressings that are applied to the human skin, and more particularly, to the field of dressings that function as fixative and/or protective (or preventive) barriers on the user's skin that may be used while the user is engaged in physical activity.

The human skin may be subject to certain types of injuries or conditions that may not limit the user's mobility. A sports enthusiast that enjoys sports such as basketball or tennis may be able to continue to play in a sporting event despite having sustained a cut, a blister or even while having a corn. Even minor surgical incisions, or minor cuts may not be sufficient to limit normal physical activity. Wound dressings are often applied with the expectation that they will stay on for up to several days. However, such dressings may be subject to stresses imposed by the wearer's activity that actually limit the stay-on time dramatically. The dressing may tend to peel off the skin as the wearer moves and the dressing rubs against the wearer's clothing.

Adhesives may be used in order to improve wear-time. However, such adhesives may irritate the skin causing pain and discomfort. For example, acrylic adhesives provide a strong bond to the skin, but are not typically used on wound dressings, particularly where the adhesive may come into contact with the wound bed. The pain and discomfort from stronger adhesives may be further aggravated during removal by the resulting irritation of the skin or by the removal of hair trapped between the skin and the bandage.

WO-A-02066087 discloses a composition suitable for use as a wound dressing comprising a polymeric matrix and absorbent particles.

EP-A-1020195 discloses a composition suitable for use as a wound dressing comprising an elastomer, a tackifier, an extender and an antioxidant.

EP-A-1020198 discloses a dressing comprising a dressing layer having a skin-contacting surface, the dressing layer comprising a material selected from hydrocolloid adhesive, silicone, hydrogel, acrylic, rubber and resin.

WO-A-0105340 discloses an ostomy appliance comprising an adhesive, flexible, skin barrier product having a hole for receiving a stoma, ureter or catheter. The adhesive and barrier zone of the skin barrier product comprises a mixture of rubbery components, starch, hydrocolloids, tackifier resins, plasticizers and a pigment.

WO-A-9954422 discloses a pressure sensitive adhesive composition for medical purposes comprising a blockcopolymer, hydrocolloids, and a tackifier resin.

The object of the invention is to obtain a dressing that may be used on the human skin for treating or preventing the formation of blisters, corns, warts, calluses, and any cut or wound that may be worn for an extended period of time, even withstanding patient mobility, without causing pain and discomfort during removal. Patients that require the use of fixed therapeutic devices (such as ostomy bags, catheters, or syringes, for example) would also benefit from the use of a fixative dressing that would allow the wearer to move and to take part in some physical activity without becoming susceptible to falling off. Since the fixative dressing would require changing from time to time, it would be advantageous to both extend the wear time to reduce the amount of changing and to ease the pain of removal.

In a first aspect of the present invention, this is achieved by a a wound dressing. The wound dressing comprises a dressing layer with a skin-contacting surface. The dressing layer comprises 20-60% by weight of a highly water-absorbable material; 5-60% by weight hot melt acrylic adhesive; 5-40% by weight tackifier; 5-30% elastomers; and 5-30% by weight extender.

In a second aspect of the present invention, the object is achieved by a composition for application to wounds. The composition comprises 20-60% by weight of a highly water-absorbable material; 5-60% by weight hot melt acrylic adhesive; 5-40% by weight tackifier; 5-30% elastomers; and 5-30% by weight extender.

In a third aspect of the present invention, the object is achieved by a fixation device having an adhesive composition. The adhesive composition comprises the composition according to the second aspect of the present invention.

The preferred embodiments regarding the wound dressing, the composition and the fixation device are described in the claims and in the following.

The details of the above-mentioned and other aspects of the present invention as well as any other embodiments will become apparent to one with skill in the art upon examination of the following figures and detailed description. It is intended that all such additional aspects, and embodiments, any advantages, features and benefits described herein or understood by one of ordinary skill in the art be included within this description, be within the scope of the invention, and be protected by the accompanying claims.

### BRIEF DESCRIPTION OF THE DRAWINGS

Presently preferred embodiments of the invention are described below in conjunction with the appended drawing figures, wherein like reference numerals refer to like elements in the various figures, and wherein:
Figure 1 represents a perspective view of a dressing according to a preferred embodiment of the present invention.
Figure 2 represents a fragmentary cross-sectional view of the device in Figure 1.
Figure 3 represents a top view of a dressing according to a preferred embodiment of the present invention configured for use as a fixation device.

### DETAILED DESCRIPTION OF THE PREFERRED EMBODIMENT

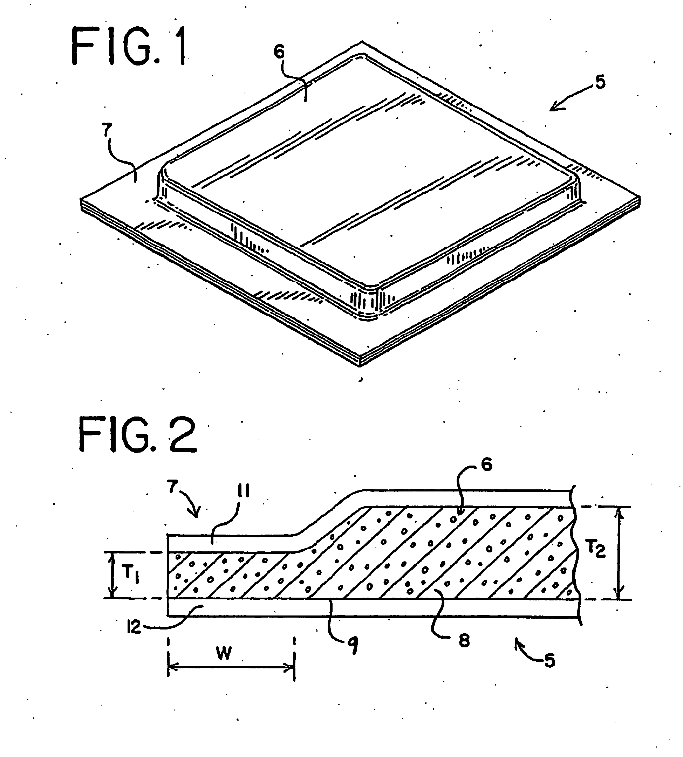

Figure 1 shows an individual dressing **5** according to a preferred embodiment of the present invention. This dressing **5** has a thick portion **6** and a thin flange portion **7.** The dressing **5** comprises a formulation that combines a hydrocolloid adhesive with acrylic to advantageously provide extended stay-on without the pain and irritation typically experienced with strong adhesives such as pure acrylic adhesives. In addition, the dressing **5** in Figure 1 may be removed easily without the pain associated with the removal of dressings that use strong adhesives.

The dressing **5** may be used to treat wounds or cuts on the human skin. The dressing **5** may also be used to treat or prevent the formation of blisters, corns, calluses, small cuts, warts, or other such conditions on the human skin. The shape of the dressing **5** in Figure 1 is rectangular, however, the dressing **5** may have any shape.

Figure 2 shows a fragmentary cross-sectional view of one example of the dressing **5** shown in Figure 1. The dressing **5** in Figure 2 includes a dressing layer **8,** a release sheet **12,** and a protective cover layer **11.** The dressing layer **8** preferably makes contact with an area of skin at a skin-contacting surface **9** and protects the skin from abrasion and exposure to infectious particles. In exemplary embodiments, the dressing **5** stays on the wound area despite the continued mobility of the patient leading to extended stay-on time. The material properties of the dressing **5** in exemplary embodiments provide unexpected stay-on times combined with a lower degree of pain and discomfort upon the removal of the dressing. The combined features of extended stay-on times and low degree of pain on removal are particularly advantageous when the dressing is applied to areas having an appreciable amount of hair.

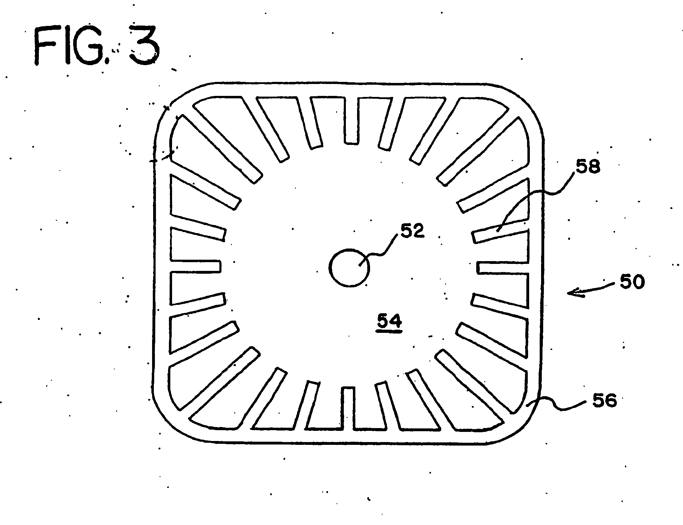

Referring to Figure 3, exemplary embodiments of the present invention may also include dressings such as a fixation device **50.** The fixation device **50** is an example of an ostomy wafer. The fixation device **50** comprises a thick portion **54,** a thin portion **56** and a hole **52** that may be used to hold an ostomy device to an opening to the body. The thick and think portions **54, 56** comprise a dressing layer similar to the dressing layer **8** described above with reference to Figure 2. The composition of the dressing layer of the fixation device of Figure 3 is described below in conjunction with the description of the dressing layer **8** of Figure 2. One of ordinary skill in the art will appreciate that the ostomy wafer depicted as the fixation device **5** is only one example of such fixation devices. Other examples include a dressings for affixing catheters, syringes, and other therapeutic devices to the body.

The material comprising the dressing layer **8** includes an adhesive to secure the dressing layer **8** to the skin. The adhesive includes a combination of hot melt acrylic adhesive and a tackifier, such as a hydrocarbon resin. This combination of hot melt acrylic and hydrocarbon resin tackifier surprisingly produces an adhesive that not only stays adhered to skin for an extended amount of time, it also comes off the skin without causing the pain and irritation normally associated with the removal of wound dressings. This is unexpected because acrylic adhesives are too sticky and therefore not commonly used in wound dressing applications at all. Their strong adhesiveness inflicts too much pain on the user upon removal. For wound dressings that cover a large area of skin and that may need to remain on the wearer for an extended amount of time, such as ostomy applications, the skin irritation caused by acrylic adhesives makes dressings with acrylics unusable.

In accordance with exemplary embodiments, the dressing layer **8** comprises:
1. 20-60% by weight of a highly water-absorbable material;
2. 5-60% by weight hot melt acrylic adhesive;
3. 5-40% by weight tackifier;
4. 5-30% elastomer; and
5. 5-30% by weight extender or plasticizer.

It is preferred that the highly water-absorbable material may be a hydrocolloid material. One example of a hydrocolloid that may be used is calcium carboxymethylcellulose ("CMC"). Others include pectin, gelatin, high molecular weight carbowax, carboxypolymethylene, polyacrylate, polyvinyl alcohol, and polyvinyl pyrrolidone.

Also the tackifier is preferably a hydrocarbon- based resin. The elastomer is preferably a styrene-olefin-styrene compound, but may also be polyisobutylene, natural rubber, silicone rubber, acrylonitrile rubber, and polyurethane rubber. The extender is preferably paraffin oil. The extender may also be a material that functions as a plasticizer in combination with the elastomer. Such plasticizers include glycerin (glycerol), sorbitol, triethylene glycol. The extender may also be mineral oil.

In one preferred embodiment, the dressing layer 8 comprises:
1. 50.5% CMC;
2. 13.5% acrylic;
3. 17% hydrocarbon tackifier (preferably Arkon P115™);
4. 12% elastomer, preferably Krayton; and
5. 8% extender, preferably paraffin oil.

For over-the-counter devices, the above quantities are preferably changed to 20% acrylic, 20% hydrocarbon tackifier, and 38% CMC. This combination provides a more tacky compound that would be better suited for high friction environments encountered with over-the-counter devices.

Although not necessary for purposes of the invention, the adhesives (*i.e.* the hydrocarbon tackifier and the acrylic) should optimally be combined such that the hydrocarbon tackifier is in equal strength per gram weight as the acrylic. Arkon P115™ and acrylic have similar tack properties per density and are therefore mixed in the same percentages. Mixing in the same percentages is not necessary, however, as different hydrocarbon tackifiers may be added to acrylic to provide the advantages of extended stay-on and relatively pain-free removal.

Exemplary embodiments of the present invention have been described. Further, the wound dressing preferably comprises a release sheet (12) and a protective cover sheet (11).
Moreover, the dressing layer has an inner hole. The preferred embodiments in connection with the wound dressing also apply to the compositions and fixation devices of the invention.

## Claims

1. A wound dressing comprising a dressing layer having a skin-contacting surface,
the dressing layer comprising:
20-60% by weight of a highly water-absorbable material;
5-60% by weight hot melt acrylic adhesive;
5-40% by weight tackifier;
5-30% elastomers;
5-30% by weight extender.

2. The wound dressing of claim 1 wherein the highly water-absorbable material is a hydrocolloid.

3. The wound dressing of claim 2 wherein the hydrocolloid is a material selected from the group consisting of: calcium carboxymethylcellulose ("CMC"), pectin, gelatin, high molecular weight carbowax, carboxypolymethylene.

4. The wound dressing of claim 1 wherein the highly water-absorbable material is a material selected from the group consisting of: polyacrylate, polyvinyl alcohol, polyvinyl pyrrolidone.

5. The wound dressing of claim 1 wherein the extender is paraffin oil.

6. The wound dressing of claim 1 wherein the extender is a material that functions as a plasticizer in combination with the elastomer.

7. The wound dressing of claim 1 wherein the tackifier is a hydrocarbon-based resin.

8. The wound dressing of claim 1 wherein the elastomer is a composition selected from the group consisting of Styrene-Olefin-Styrene, polyisobutylene, natural rubber, silicone rubber, acrylonitrile rubber, and polyurethane rubber.

9. The wound dressing of claim 1 wherein the dressing layer comprises:
50.5% CMC;
13.5% acrylic adhesive;
17% tackifier;
12% elastomer; and
8% extender.

10. The wound dressing of claim 1 wherein the dressing layer comprises:
38% CMC;
20% acrylic adhesive; and
20% tackifier.

11. The wound dressing of claim 1 further comprising:
a release sheet, and
a protective cover layer.

12. The wound dressing of claim 1 wherein the dressing layer has an inner hole.

13. A composition for application to wounds in a wound dressing comprising:
20-60% by weight of a highly water-absorbable material;
5-60% by weight hot melt acrylic adhesive;
5-40% by weight tackifier;
5-30% elastomers;
5-30% by weight extender.

14. The composition of claim 13 wherein the highly water-absorbable material is a hydrocolloid.

15. The composition of claim 14 wherein the hydrocolloid is a material selected from the group consisting of: calcium carboxymethylcellulose ("CMC"), pectin, gelatin, high molecular weight carbowax, carboxypolymethylene.

16. The composition of claim 13 wherein the highly water-absorbable material is a material selected from the group consisting of: polyacrylate, polyvinyl alcohol, polyvinyl pyrrolidone.

17. The composition of claim 13 wherein the extender is a paraffin oil.

18. The composition of claim 13 wherein the extender is a material that functions as a plasticizer in combination with the elastomer.

19. The composition of claim 13 wherein the tackifier is a hydrocarbon-based resin.

20. The composition of claim 13 wherein the elastomer is a composition selected from the group consisting of Styrene-Olefin-Styrene, polyisobutylene, natural rubber, silicone rubber, acrylonitrile rubber, and polyurethane rubber.

21. A fixation device having an adhesive composition comprising the composition according to claim 13.

22. The fixation device of claim 21 wherein the highly water-absorbable material is a hydrocolloid.

23. The fixation device of claim 22 wherein the hydrocolloid is a material selected from the group consisting of: calcium carboxymethylcellulose ("CMC"), pectin, gelatin, high molecular weight carbowax, carboxypolymethylene.

24. The fixation device of claim 21 wherein the highly water-absorbable material is a material selected from the group consisting of: polyacrylate, polyvinyl alcohol, polyvinyl pyrrolidone.

25. The fixation device of claim 21 wherein the extender is paraffin oil.

26. The fixation device of claim 21 wherein the extender is a material that functions as a plasticizer in combination with the elastomer.

27. The fixation device of claim 21 wherein the tackifier is a hydrocarbon-based resin.

28. The fixation device of claim 21 wherein the elastomer is a composition selected from the group consisting of Styrene-Olefin-Styrene, polyisobutylene, natural rubber, silicone rubber, acrylonitrile rubber, and polyurethane rubber.

29. The fixation device of claim 21 wherein the dressing layer comprises:
50.5% CMC;
13.5% acrylic adhesive;
17% tackifier;
12% elastomer; and
8% extender.

30. The fixation device of claim 21 wherein the dressing layer comprises:
38% CMC;
20% acrylic adhesive; and
20% hydrocarbon tackifier.

31. The fixation device of claim 21 further comprising:
a release sheet, and
a protective cover layer.

## Patentansprüche

1. Wundverband, umfassend eine Verbandsschicht mit einer mit der Haut in Kontakt kommenden Oberfläche, wobei die Verbandsschicht umfasst:
20 - 60 Gew.% eines in hohem Maße wasserabsorptionsfähigen Materials;
5 - 60 Gew.% Acrylschmelzklebstoff;
5 - 40 Gew.% Klebrigmacher;
5 - 30 Gew.% Elastomere;
5 - 30 Gew.% Füllstoff.

2. Wundverband nach Anspruch 1, wobei das in hohem Maße wasserabsorptionsfähige Material ein Hydrokolloid ist.

3. Wundverband nach Anspruch 2, wobei das Hydrokolloid ein Material ist, ausgewählt aus der Gruppe, bestehend aus: Calciumcarboxymethylcellulose (CMC), Pektin, Gelatine, hoch molekularem Carbowachs, Carboxypolymethylen.

4. Wundverband nach Anspruch 1, wobei das in hohem Maße wasserabsorptionsfähige Material ausgewählt ist aus der Gruppe, bestehend aus: Polyacrylat, Polyvinylalkohol, Polyvinylpyrrolidon.

5. Wundverband nach Anspruch 1, wobei der Füllstoff Paraffinöl ist.

6. Wundverband nach Anspruch 1, wobei der Füllstoff ein Material ist, das in Kombination mit dem Elastomer als Weichmacher fungiert.

7. Wundverband nach Anspruch 1, wobei der Klebrigmacher ein kohlenwasserstoffbasiertes Harz ist.

8. Wundverband nach Anspruch 1, wobei das Elastomer eine Zusammensetzung ist, ausgewählt aus der Gruppe, bestehend aus Styrol-Olefin-Styrol, Polyisobutylen, Naturkautschuk, Silikonkautschuk, Acrylnitrilkautschuk und Polyurethankautschuk.

9. Wundverband nach Anspruch 1, wobei die Verbandsschicht umfasst:
50,5% CMC;
13,5% Acrylklebstoff;
17% Klebrigmacher;
12% Elastomer; und
8% Füllstoff.

10. Wundverband nach Anspruch 1, wobei die Verbandsschicht umfasst:
38% CMC;
20% Acrylklebstoff; und
20% Klebrigmacher.

11. Wundverband nach Anspruch 1, weiterhin umfassend:
eine Trennfolie, und
eine schützende Deckschicht.

12. Wundverband nach Anspruch 1, wobei die Verbandsschicht ein Innenloch hat.

13. Zusammensetzung zur Anwendung an Wunden in einem Wundverband umfassend:
20 - 60 Gew.% eines in hohem Maße wasserabsorptionsfähigen Materials;
5 - 60 Gew.% Acrylschmelzklebstoff;
5 - 40 Gew.% Klebrigmacher;
5 - 30 Gew.% Elastomere;
5 - 30 Gew.% Füllstoff.

14. Zusammensetzung nach Anspruch 13, wobei das in hohem Maße wasserabsorptionsfähige Material ein Hydrokolloid ist.

15. Zusammensetzung nach Anspruch 14, wobei das Hydrokolloid ein Material ist, ausgewählt aus der Gruppe, bestehend aus: Calciumcarboxymethylcellulose (CMC), Pektin, Gelatine, hochmolekularem Carbowachs, Carboxypolymethylen.

16. Zusammensetzung nach Anspruch 13, wobei das in hohem Maße wasserabsorptionsfähige Material ein Material ist, ausgewählt aus der Gruppe, bestehend aus: Polyacrylat, Polyvinylalkohol, Polyvinylpyrrolidon.

17. Zusammensetzung nach Anspruch 13, wobei der Füllstoff Paraffinöl ist.

18. Zusammensetzung nach Anspruch 13, wobei der Füllstoff ein Material ist, das in Kombination mit dem Elastomer als Weichmacher fungiert.

19. Zusammensetzung nach Anspruch 13, wobei der Klebrigmacher ein kohlenwasserstoffbasiertes Harz ist.

20. Zusammensetzung nach Anspruch 13, wobei das Elastomer eine Zusammensetzung ist, ausgewählt aus der Gruppe, bestehend aus Styrol-Olefin-Styrol, Polyisobutylen, Naturkautschuk, Silikonkautschuk, Acrylnitrilkautschuk und Polyurethankautschuk.

21. Fixiervorrichtung mit einer haftenden Zusammensetzung, umfassend die Zusammensetzung nach Anspruch 13.

22. Fixiervorrichtung nach Anspruch 21, wobei das in hohem Maße wasserabsorptionsfähige Material ein Hydrokolloid ist.

23. Fixiervorrichtung nach Anspruch 22, wobei das Hydrokolloid ein Material ist, ausgewählt aus der Gruppe, bestehend aus: Calciumcarboxymethylcellulose (CMC), Pektin, Gelatine, hochmolekularem Carbowachs, Carboxypolymethylen.

24. Fixiervorrichtung nach Anspruch 21, wobei das in hohem Maße wasserabsorptionsfähige Material ein Material ist, ausgewählt aus der Gruppe, bestehend aus: Polyacrylat, Polyvinylalkohol, Polyvinylpyrrolidon.

25. Fixiervorrichtung nach Anspruch 21, wobei der Füllstoff Paraffinöl ist.

26. Fixiervorrichtung nach Anspruch 21, wobei der Füllstoff ein Material ist, das in Kombination mit dem Elastomer als Weichmacher fungiert.

27. Fixiervorrichtung nach Anspruch 21, wobei der Klebrigmacher ein kohlenwasserstoffbasiertes Harz ist.

28. Fixiervorrichtung nach Anspruch 21, wobei das Elastomer eine Zusammensetzung ist, ausgewählt aus der Gruppe, bestehend aus Styrol-Olefin-Stryrol, Polyisobutylen, Naturkautschuk, Silikonkautschuk, Acrylnitrilkautschuk, und Polyurethankautschuk.

29. Fixiervorrichtung nach Anspruch 21, wobei die Verbandsschicht umfasst:
50,5% CMC;
13,5% Acrylklebstoff;
17% Klebrigmacher;
12% Elastomer; und
8% Füllstoff.

30. Fixiervorrichtung nach Anspruch 21, wobei die Verbandsschicht umfasst:
38% CMC;
20% Acrylklebstoff; und
20% Kohlenwasserstoffklebrigmacher

31. Fixiervorrichtung nach Anspruch 21, weiterhin umfassend:
eine Trennfolie, und
eine schützende Deckschicht.

## Revendications

1. Pansement comprenant une couche formant compresse ayant une surface en contact avec la peau, la couche formant compresse comprenant :
20 à 60 % en poids d'un matériau ayant un fort pouvoir d'absorption de l'eau ;
5 à 60 % en poids d'un adhésif acrylique thermofusible ;
5 à 40 % en poids d'agent poisseux ;
5 à 30 % d'élastomères ;
5 à 30 % en poids d'agent d'extension.

2. Pansement selon la revendication 1, dans lequel le matériau ayant un fort pouvoir d'absorption de l'eau est un hydrocolloïde.

3. Pansement selon la revendication 2, dans lequel l'hydrocolloïde est un matériau choisi dans l'ensemble constitué par la carboxyméthylcellulose calcique ("CMC"), la pectine, la gélatine, le carbowax de haute masse moléculaire, le carboxypolyméthylène.

4. Pansement selon la revendication 1, dans lequel le matériau ayant un fort pouvoir d'absorption de l'eau est un matériau choisi dans l'ensemble constitué par le polyacrylate, le poly(alcool vinylique), la polyvinylpyrrolidone.

5. Pansement selon la revendication 1, dans lequel l'agent d'extension est une huile de paraffine.

6. Pansement selon la revendication 1, dans lequel l'agent d'extension est un matériau qui fonctionne comme un plastifiant en combinaison avec l'élastomère.

7. Pansement selon la revendication 1, dans lequel l'agent poisseux est une résine hydrocarbonée.

8. Pansement selon la revendication 1, dans lequel l'élastomère est une composition choisie dans l'ensemble constitué par le styrène/oléfine/styrène, le polyisobutylène, le caoutchouc naturel, le caoutchouc siliconé, le caoutchouc d'acrylonitrile, et le caoutchouc de polyuréthane.

9. Pansement selon la revendication 1, dans lequel la couche formant compresse comprend :
50,5 % de CMC ;
13,5 % d'adhésif acrylique ;
17 % d'agent poisseux ;
12 % d'élastomère ; et
8 % d'agent d'extension.

10. Pansement selon la revendication 1, dans lequel la couche formant compresse comprend :
38 % de CMC ;
20 % d'adhésif acrylique ; et
20 % d'agent poisseux.

11. Pansement selon la revendication 1, comprenant en outre :
une feuille amovible, et
une couche de revêtement protectrice.

12. Pansement selon la revendication 1, dans lequel la couche formant compresse a un trou intérieur.

13. Composition pour application à des blessures dans un pansement, comprenant :
20 à 60 % en poids d'un matériau ayant un fort pouvoir d'absorption de l'eau ;
5 à 60 % en poids d'un adhésif acrylique thermofusible ;
5 à 40 % en poids d'agent poisseux ;
5 à 30 % d'élastomères ;
5 à 30 % en poids d'agent d'extension.

14. Composition selon la revendication 13, dans laquelle le matériau ayant un fort pouvoir d'absorption de l'eau est un hydrocolloïde.

15. Composition selon la revendication 14, dans laquelle l'hydrocolloïde est un matériau choisi dans l'ensemble constitué par la carboxyméthylcellulose calcique ("CMC"), la pectine, la gélatine, le carbowax de haute masse moléculaire, le carboxypolyméthylène.

16. Composition selon la revendication 13, dans laquelle le matériau ayant un fort pouvoir d'absorption de l'eau est un matériau choisi dans l'ensemble constitué par le polyacrylate, le poly(alcool vinylique), la polyvinylpyrrolidone.

17. Composition selon la revendication 13, dans laquelle l'agent d'extension est une huile de paraffine.

18. Composition selon la revendication 13, dans laquelle l'agent d'extension est un matériau qui fonctionne comme un plastifiant en combinaison avec l'élastomère.

19. Composition selon la revendication 13, dans laquelle l'agent poisseux est une résine hydrocarbonée.

20. Composition selon la revendication 13, dans laquelle l'élastomère est une composition choisie dans l'ensemble constitué par le styrène/oléfine/styrène, le polyisobutylène, le caoutchouc naturel, le caoutchouc siliconé, le caoutchouc d'acrylonitrile, et le caoutchouc de polyuréthane.

21. Dispositif de fixation ayant une composition adhésive comprenant la composition selon la revendication 13.

22. Dispositif de fixation selon la revendication 21, dans lequel le matériau ayant un fort pouvoir d'absorption de l'eau est un hydrocolloïde.

23. Dispositif de fixation selon la revendication 22, dans lequel l'hydrocolloïde est un matériau choisi dans l'ensemble constitué par la carboxyméthylcellulose calcique ("CMC"), la pectine, la gélatine, le carbowax de haute masse moléculaire, le carboxypolyméthylène.

24. Dispositif de fixation selon la revendication 21, dans lequel le matériau ayant un fort pouvoir d'absorption de l'eau est un matériau choisi dans l'ensemble constitué par le polyacrylate, le poly(alcool vinylique), la polyvinylpyrrolidone.

25. Dispositif de fixation selon la revendication 21, dans lequel l'agent d'extension est une huile de paraffine.

26. Dispositif de fixation selon la revendication 21, dans lequel l'agent d'extension est un matériau qui fonctionne comme un plastifiant en combinaison avec l'élastomère.

27. Dispositif de fixation selon la revendication 21, dans lequel l'agent de poisseux est une résine hydrocarbonée.

28. Dispositif de fixation selon la revendication 21, dans lequel l'élastomère est une composition choisie dans l'ensemble constitué par le styrène/oléfine/styrène, le polyisobutylène, le caoutchouc naturel, le caoutchouc siliconé, le caoutchouc d'acrylonitrile, et le caoutchouc de polyuréthane.

29. Dispositif de fixation selon la revendication 21, dans lequel la couche formant compresse comprend :
50,5 % de CMC ;
13,5 % d'adhésif acrylique ;
17 % d'agent poisseux ;
12 % d'élastomère ; et
8 % d'agent d'extension.

30. Dispositif de fixation selon la revendication 21, dans lequel la couche formant compresse comprend :
38 % de CMC ;
20 % d'adhésif acrylique ; et
20 % d'agent poisseux hydrocarboné.

31. Dispositif de fixation selon la revendication 1, comprenant en outré :
une feuille amovible, et
une couche de revêtement protectrice.
